# EUROPEAN PATENT SPECIFICATION

(11) **EP 3 197 427 B1**
(45) Date of publication and mention of the grant of the patent: **24.02.2021**
(21) Application number: 15766538.1
(22) Date of filing: 22.09.2015
(51) Int. Cl.: A61K 8/37, A61K 8/55, A61Q 19/00, A61K 8/06, A61K 8/39

(54) **O/W EMULSIONS**
O/W-EMULSIONEN
ÉMULSIONS H/E

(30) Priority: 26.09.2014 EP 14186575
(43) Date of publication of application: 02.08.2017
(73) Proprietor: DSM IP Assets B.V., 6411 TE Heerlen (NL)
(72) Inventor: MENDROK-EDINGER, Christine, CH-4303 Kaiseraugst (CH); SAECKER, Christine, CH-4303 Kaiseraugst (CH); HECKER, Karina, CH-4303 Kaiseraugst (CH)
(74) Representative: Berg, Katja
(86) International application number: PCT/EP2015/071754
(87) International publication number: WO 2016/046210

(56) References cited:
- WO-A2-2012/062755
- DE-A1-102011 077 060
- US-A1- 2006 171 913
- US-A1- 2007 178 144
- US-A1- 2010 190 740
- DATABASE GNPD [Online] MINTEL; 1 November 2013 (2013-11-01), "Nourishing Body Lotion", XP002739193, Database accession no. 2213956
- DATABASE GNPD [Online] MINTEL; 1 February 2013 (2013-02-01), "Vine Body Butter", XP002739194, Database accession no. 2007698
- Clariant: "Emulsifiers for Personal Care Applications", , 16 February 2009 (2009-02-16), pages 1-40, XP055187139, Retrieved from the Internet: URL:http://www.essentialingredients.com/pd f/ClariantEmulsifiers.pdf [retrieved on 2015-05-04]

## Description

The present invention relates to topical compositions in the form of an oil-in-water (O/W) emulsion comprising an oil phase dispersed in an aqueous phase in the presence of a polyglyceryl fatty ester surfactant, characterized in that the topical composition further comprises a phosphate ester co-surfactant. Furthermore, the invention relates to the use of a phosphate ester surfactant to reduce the viscosity of a topical composition in the form of an O/W emulsion comprising an oil phase dispersed in an aqueous phase in the presence of a polyglyceryl fatty ester surfactant.

It is generally known that O/W emulsions with low viscosity often exhibit a stability problem upon storage, in particular at elevated temperatures. Thus, there is an ongoing need for agents that are able to reduce the viscosity O/W-emulsions without affecting the storage stability.

US 2007/178144 discloses discloses prolonged-stability, low-viscosity, fine O/W emulsion comprising a specific emulsifier mixture consisting of polyglyceryl-3 laurate and potassium cetyl phosphate.

DE 10 2011 077060 discloses topical O/W emulsion comprising less than 6 wt.-% of polyglyceryl-10 stearate.

Surprisingly it has been found that the addition of a phosphate ester surfactant to a topical composition in the form of an oil-in-water (O/W) emulsion comprising an oil phase dispersed in an aqueous phase in the presence of a polyglyceryl fatty ester surfactant results in a reduction of the viscosity without affecting the long term stability such as in particular the thermal stability.

Thus, in one embodiment, the present invention relates to topical compositions in the form of an oil-in-water (O/W) emulsion comprising an oil phase dispersed in an aqueous phase in the presence of a polyglyceryl fatty ester surfactant, characterized in that the topical composition further comprises a phosphate ester co-surfactant according to claim 1.

In a further embodiment the invention relates to the use of a phosphate ester surfactant to reduce the viscosity of a topical composition in the form of an O/W emulsion comprising an oil phase dispersed in an aqueous phase in the presence of a polyglyceryl fatty ester surfactant according to claim 11.

In another embodiment the invention relates to a method for reducing the viscosity of a topical composition in the form of an oil-in-water (O/W) emulsion comprising an oil phase dispersed in an aqueous phase in the presence of a polyglyceryl fatty ester surfactant, said method comprising the addition of a phosphate ester co-surfactant according to claim 12.

In a further embodiment the invention relates to a method of preserving the long-term thermal storage stability of a topical composition in the form of an oil-in-water (O/W) emulsion comprising an oil phase dispersed in an aqueous phase in the presence of a polyglyceryl fatty ester surfactant, said method comprising the addition of a phosphate ester co-surfactant according to claim 13.

The amount of the polyglyceryl fatty ester surfactant in the topical composition according to the present invention is preferably selected in the range of 0.1 to 10 wt.-%, preferably in the range of 0.3 to 5 wt.-%, most preferably in the range of 0.5 to 3 wt.-% based on the total weight of the topical composition.

The amount of the phosphate ester co-surfactant in the topical composition according to the present invention is preferably selected in the range of 0.01 to 3 wt.-%, preferably in the range of 0.05 to 2 wt.-%, most preferably in the range of 0.1 to 1 wt.-% based on the total weight of the topical composition.

Preferably in all embodiments of the invention the wt.-ratio between the polyglyceryl fatty ester surfactant and the phosphate ester co-surfactant is additionally selected in the range of 8:1 to 1:1, preferably in the range of 6:1 to 2:1, most preferably in the range of 5:1 to 3:1 such as in the range of 4.5:1 to 3.5:1.

Particularly suitable in all embodiments of the invention are phosphate esters surfactants of formula (I) 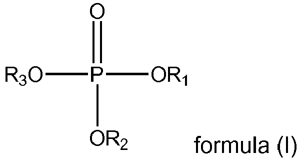 wherein
R₁, R₂ and R₃ are independently of each other hydrogen, C₁₋₂₂ alkyl, or a C₁₋₂₂ alkoxylated alkyl having 2 to 25 moles ethylene oxide,
with the proviso that at least one of R₁, R₂ and R₃ is an alkyl or an alkoxylated alkyl having at least 6 alkyl carbons in said alkyl or alkoxylated alkyl group.

Preferably R₁, R₂ and R₃ are independently of each other hydrogen, C₁₂₋₁₈ alkyl, or a C₁₂₋₂₈ alkoxylated alkyl having 2 to 12 moles ethylene oxide.

Monoesters in which R₁ and R₂ are hydrogen and R₃ is selected from C₁₂₋₁₈ alkyl and alkoxylated fatty alcohols having from 10 to 18 carbons and 2 to 12 moles ethylene oxide are preferred. Among the preferred phosphate ester surfactants are C₈-₁₀ Alkyl Ethyl Phosphate, C₉₋₁₅ Alkyl Phosphate, Ceteareth-2 Phosphate, Ceteareth-5 Phosphate, Ceteth-8 Phosphate, Ceteth-10 Phosphate, Cetyl Phosphate, Dicetyl Phosphate, C6-10 Pareth-4 Phosphate, C₁₂₋₁₅ Pareth-2 Phosphate, C₁₂₋₁₅ Pareth-3 Phosphate, DEA-Ceteareth-2 Phosphate, DEA-Cetyl Phosphate, DEA-Oleth-3 Phosphate, Potassium Cetyl Phosphate, Deceth-4 Phosphate, Deceth-6 Phosphate and Trilaureth-4 Phosphate. Most preferably, in all embodiments of the present invention, potassium cetyl phosphate e.g. commercially available as AMPHISOL® K at DSM Nutritional Products Ltd Kaiseraugst is used.

The term 'polyglyceryl fatty ester' as used according to the present invention refers to compounds which are obtainable by esterification of fatty acids to one or several hydroxyl groups of polyglycerol. Such polyglyceryl fatty esters are widely used as surfactants in the cosmetic industry and well known to a person skilled in the art.

The term 'fatty', as used herein, preferably refers to a hydrocarbon chain having 12-22 carbon atoms (C₁₂₋₂₂). The chain may be straight or branched and may be saturated or unsaturated (typically one or two double bonds in the chain).

Suitable polyglycerol fatty ester surfactants are the ones of formula (II) 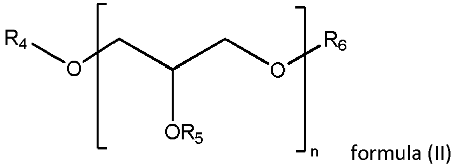 wherein
R₄, R₅ and R₆ are independently of each other hydrogen, a stearoyl and/ or an isostearoyl group with the proviso that at least one of R₄, R₅ and R₆ is a stearoyl- and/ or an isostearoyl-group and n is an integer selected from the group of 2 to 10, most preferably from 3 to 10.

It is further preferred when the polyglyceryl fatty ester surfactants of formula (II) are mono- or diesters of either stearic acid or isostearic acid. Most preferred polyglycerol fatty ester surfactants according to the present invention are polyglyceryl-3-stearate (also known as triglyceryl monostearate; CAS: 27321-72-8 or 37349-34-1 (generic); HLB approx. 9) which is e.g. available as Dermofeel PS by Dr. Straetmans, polyglyceryl-10-stearate (also known as decaglyceryl monostearate; CAS: 79777-30-3 or 9009-32-9 (generic); HLB approx. 12) which is e.g. available as Polyaldo 10-1-S KFG by Lonza, polyglyceryl-2-diisostearate (also known as diglyceryl diisostearate; CAS: 67938-21-0 or 63705-03-3 (generic); HLB approx. 6) which is e.g. available as Salacos 42V by Nisshin, polyglyceryl-6-distearate (also known as hexaglyceryl monostearate; CAS: 34424-97-0 or 9009-32-9 (generic); HLB approx. 9) which is e.g. available as Plurol Stearique WL 1009 by Gattefosse, polyglyceryl-10 diisostearate (also known as decaglyceryl diisostearate; CAS: 102033-55-6 or 63705-03-3 (generic); HLB approx. 11) which is e.g. available as Nikkol Decaglyn 2-IS by Nikko, as well as a blend of polyglyceryl-6-distearate and polyglyceryl-3-beeswax with jojoba esters and cetyl alcohol which is e.g. commercially available as Emulium® Mellifera (INCI Polyglyceryl-6 Distearate (and) Jojoba Esters (and) Polyglyceryl-3 Beeswax (and) Cetyl Alcohol) by Gattefosse (HLB approx. 10.5).

The polyglyceryl fatty ester surfactant used in the topical compositions according to the present invention also ideally will have an HLB of at least 5 or greater. Preferably the polyglyceryl fatty ester surfactant used in the topical compositions according to the present invention exhibit an HLB selected in the range of 6 to 15, most preferably in the range of 9 to 14.

In a particular preferred embodiment the topical compositions according to the present invention contain - next to the polyglyceryl fatty ester surfactant(s) and the phosphate esters co-surfactant(s) - additional surfactants and/ or co-surfactants. Most preferably such additional surfactants and/ or co-surfactants are selected from the group consisting of fatty alcohols such as cetyl alcohol, stearyl alcohol, cetearyl alcohol, behenyl alcohol and the like and glyceryl stearate.

The viscosity reduction achieved by the phosphate ester surfactant is preferably at least 30%, preferably at least 30%, most preferably in the range of about 50% measured at 20°C [in mPas at 22°C; Brookfield Viscosimeter DV-II Pro].

Particular preferred topical compositions according to the present invention exhibit a viscosity of 50'000 mPas or less, such as preferably a viscosity selected in the range of 100 to 30'000 mPas and most preferably selected in the range of 100 to 25'000 mPas after 10 days of storage at RT.

The topical compositions according to the present invention can be in the form of a serum, milk, emulsion spray, lotion or cream, and they are prepared according to the usual methods. The compositions which are the subject-matter of the present invention are intended for topical application and can in particular constitute dermatological or cosmetic compositions which are, for example, intended for the protection of human skin against the adverse effects of UV radiation (antiwrinkle, anti-ageing, moisturizing, anti-sun protection and the like) or intended to moisturize and soothe the skin.

According to an advantageous embodiment of the invention the compositions constitute cosmetic composition which are intended for topical application to the skin.

The topical compositions of the invention preferably further contain usual cosmetic adjuvants and additives such as preservatives/antioxidants, fatty substances/ oils, organic solvents, silicones, thickeners, softeners, emulsifiers, antifoaming agents, aesthetic components such as fragrances, surfactants, fillers, sequestering agents, anionic, cationic, nonionic or amphoteric polymers, propellants, acidifying or basifying agents, dyes, colorants, abrasives, absorbents, essential oils, sensory modifiers, astringents, or any other ingredients usually formulated into cosmetic compositions. Such cosmetic adjuvants and additives commonly used in the skin care industry, which are suitable for use in the compositions of the present invention are e.g. described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

In accordance with the present invention, the topical compositions according to the invention may further comprise one or more skin active or protective agent such as skin lightening agents, agents for tanning prevention and/ or treatment of hyperpigmentation; skin-tanning agents; agents for the prevention or reduction of acne, anti-ageing agents, anti-inflammatory agents; moisturizers; anti-cellulite agents, slimming agents (e.g. phytanic acid), soothing agents, agents to improve the skin elasticity and skin barrier as well as UV-filter substances. The necessary amounts of such skin active or protective agents can, based on the desired product, easily be determined by the skilled person. The additional agents can either be added to the oil phase, the aqueous phase or separately as deemed appropriate. The mode of addition can easily be adapted by a person skilled in the art.

Particular suitable skin-lightening, tanning prevention and/ or treatment of hyperpigmentation agents are in particular ascorbic acid and its derivatives such as sodium ascorbyl glycoside, sodium ascorbyl phosphate and magnesium ascorbyl phosphate, biotin, niacinamide, and/ or alpha arbutin.

Particular suitable skin-tanning agents are dihydroxyacetone and/ or erythrulose.

Particular suitable anti-ageing agents encompass natural extracts or peptides such as in particular SYN®-COLL or SYN®-AKE which are commercially available at DSM Nutritional Products Ltd.

Particular suitable UV-filter substances to be incorporated into the topical compositions according to the present invention encompass in particular the commercially available and widely used UV-filter substances octocrylene (PARSOL® 340), 4-methyl benzylidene camphor (PARSOL® 5000), ethylhexyl methoxycinnamate (PARSOL® MCX), ethylhexyl triazone (Uvinul® T-150), diethylhexyl butamido triazone (Uvasorb® HEB), 2,2'-methylene-bis-(6-(2H-benzotriazole-2-yl)-4-(1,1,3,3,-tetramethylbutyl)-phenol (Tinosorb® M), bis-ethylhexyl-oxyphenol methoxyphenyl triazine (Tinosorb® S), 2,2-(1,4-phenylene)bis-(1H-benzimidazol-4,6-disulfonic acid (NeoHeliopan® AP), 2-(4-Diethylamino-2-hydroxy-benzoyl)-benzoic acid hexylester (Uvinul® A plus), 1,1'-(1,4-piperazinediyl)bis[1-[2-[4-(diethylamino)-2-hydroxybenzoyl]phenyl]-methanone (CAS No 919803-06-8), polysilicone-15 (PARSOL® SLX), 2-phenyl benzimidazole sulfonic acid (PARSOL® HS), ethylhexyl salicylate (PARSOL® EHS), homomenthyl salicylate (PARSOL® HMS), Benzophenone-3 (Uvinul® M 40), Benzophenone-4 (Uvinul® MS 40), Butyl Methoxydibenzoyl Methane (PARSOL® 1789), Terephtalidene dicampher Sulfonic Acid (Mexoryl® SX), Drometrizole Trisiloxane (Mexoryl® XL), Tris-Biphenyl Triazine (nano) (Tinosorb® A2B), microfine zinc oxide (coated or uncoated) or titanium dioxide (coated or uncoated) such as in particular PARSOL® TX, as well as mixtures thereof.

Generally, the amount of each UV-filter substance in the topical compositions according to the invention is selected in the range of about 0.1 to 15 wt.-%, preferably in the range of about 0.5 to 10 wt.-%, most preferably in the range of about 1 to 8 wt.-% with respect to the total weigh of the topical composition.

The total amount of UV-filter substances in the topical compositions according to the invention is preferably selected in the range from 0.1 to 35 wt.-%, preferably in the range from 1 to 15 wt.-%, most preferably in the range from 5 to 35 wt.-% based on the total weight of the topical composition.

The skin active or protective agents useful herein can in some instances provide more than one benefit or operate via more than one mode of action.

The necessary amounts of the cosmetic and dermatological adjuvants and additives can - based on the desired product - easily be chosen by a skilled person in this field and will be illustrated in the examples, without being limited hereto.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

The topical compositions according to the invention in general have a pH in the range of 3 to 10, preferably a pH in the range of 4 to 8 and most preferably a pH in the range of 4.5 to 7.5. The pH can easily be adjusted as desired with suitable acids such as e.g. citric acid or bases such as sodium hydroxide (e.g. as aqueous solution), potassium hydroxide, triethanolamine (TEA Care), tromethamine (Trizma Base) and aminomethyl propanol (AMP-Ultra PC 2000) according to standard methods in the art.

The amount of the topical composition to be applied to the skin is not critical and can easily be adjusted by a person skilled in the art. Preferably the amount is selected in the range of 0.1 -3 mg/ cm² skin, such as preferably in the range of 0.1 to 2 mg/ cm² skin and most preferably in the range of 0.5 to 2 wt.-% / cm².

The following examples are provided to further illustrate the compositions and effects of the present invention. These examples are illustrative only and are not intended to limit the scope of the invention in anyway.

### Example

The cosmetic formulations have been prepared according standard procedures.
Dissolve the water soluble ingredients in water and heat it up to 80°C. Combine the oil soluble ingredients and heat it up to 80°C. Combine the two phases under stirring and homogenize with an Ultra Turrax for 1 minute at 13000rpm. Cool down under stirring. Add preservative and neutralizing agent below 40°C under stirring.
The initial viscosity of a 100g sample of each formulation has been measured at 20°C with a Brookfield Viscosimeter DV-II Pro in a 100ml beaker 24hours after preparation of the formulation using spindle S5 at 2.5rpm.
The formulations are stored at RT (about 22°C) and 40°C. The viscosities have consecutively been measured again as indicated in table 1.

The results in table 1 illustrate that in the presence of potassium cetyl phosphate as co-surfactant the viscosity surprisingly increases during storage at 40°C (compared to storage at RT), which indicates an excellent thermal stability. In contrast, in the absence of potassium cetyl phosphate the viscosity decreases during storage at 40°C (compared to storage at RT) as expected by a person skilled in the art which indicates the thermal instability.

**Table 1**

| | A | B | C (Control) | D | E | F (Control) |
|---|---|---|---|---|---|---|
| Water | Ad 100 | Ad 100 | Ad 100 | Ad 100 | Ad 100 | Ad 100 |
| Glycerin | 5.00 | 5.00 | 5.00 | 5.00 | 5.00 | 5.00 |
| Disodium EDTA | 0.10 | 0.10 | 0.10 | 0.10 | 0.10 | 0.10 |
| Xanthan Gum | 0.15 | 0.15 | 0.15 | 0.15 | 0.15 | 0.15 |
| Potassium Cetyl Phosphate | 0.50 | 0.50 | - | 0.50 | 0.50 | - |
| Butyl Methoxydibenzoylmethane | 4.00 | 4.00 | 4.00 | 4.00 | 4.00 | 4.00 |
| Octocrylene | 10.00 | 10.00 | 10.00 | 10.00 | 10.00 | 10.00 |
| Homosalate | 10.00 | 10.00 | 10.00 | 10.00 | 10.00 | 10.00 |
| Titanium Dioxide, Silica, Dimethicone | 3.00 | 3.00 | 3.00 | 3.00 | 3.00 | 3.00 |
| Polyglyceryl-3 Stearate | 2.00 | 1.50 | 2.00 | | | |
| Polyglyceryl-6 Distearate, Jojoba Esters, Polyglyceryl-3 Beeswax, Cetyl Alcohol | | | | 2.00 | 1.50 | 2.00 |
| Cetearyl Alcohol | 2.00 | 2.00 | 2.00 | 2.00 | 2.00 | 2.00 |
| Butylene Glycol Dicaprylate/Dicaprate | 4.00 | 4.00 | 4.00 | 4.00 | 4.00 | 4.00 |
| Dicaprylyl Carbonate | 3.00 | 3.00 | 3.00 | 3.00 | 3.00 | 3.00 |
| Phenoxyethanol, Ethylhexylglycerin | 1.00 | 1.00 | 1.00 | 1.00 | 1.00 | 1.00 |
| Tromethamine | 0.25 | 0.25 | 0.25 | 0.25 | 0.25 | 0.25 |
| | | | | | | |
| Viscosity [mPas]/ initial | 46250 | 41120 | 72000 | 38560 | 48480 | 51500 |
| Viscosity reduction versus control | -36% | -43% | | -25% | -6% | |
| Viscosity [mPas]/ 10 days/ RT | 20240 | 14400 | 26000 | 18840 | 24640 | 20000 |
| Viscosity [mPas]/ 10 days/ 40°C | 24800 | 20000 | 17280 | 22600 | 26580 | 14000 |

## Claims

1. Topical composition in the form of an oil-in-water (O/W) emulsion comprising an oil phase dispersed in an aqueous phase in the presence of a polyglyceryl fatty ester surfactant and a phosphate ester co-surfactant, **characterized in that** the polyglyceryl fatty ester surfactant is a mono-ester of stearic acid or isostearic acid of formula (II) 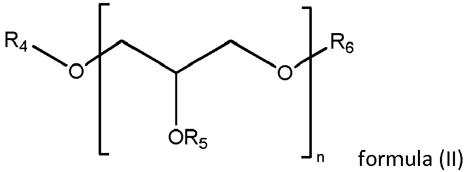 wherein
R₄, R₅ and R₆ are independently of each other hydrogen, a stearoyl and/ or an isostearoy group with the proviso that one of R₄, R₅ and R₆ is a stearoyl- and/ or an isostearoyl-group and the other ones are hydrogen and n is an integer selected from 2 to 10 and wherein the wt.-ratio between the polyglyceryl fatty ester surfactant and the phosphate ester co-surfactant is selected in the range of 8:1 to 1:1.

2. The topical composition according to claim 1, **characterized in that** the amount of the polyglyceryl fatty ester surfactant is selected in the range of 0.1 to 10 wt.-%, preferably in the range of 0.3 to 5 wt.-%, most preferably in the range of 0.5 to 3 wt.-% based on the total weight of the topical composition.

3. The topical composition according to claim 1 or 2, **characterized in that** the amount of the phosphate ester co-surfactant is selected in the range of 0.01 to 3 wt.-%, preferably in the range of 0.05 to 2 wt.-%, most preferably in the range of 0.1 to 1 wt.-% based on the total weight of the topical composition.

4. The topical composition according to any one of claims 1 to 3, **characterized in that** the wt.-ratio between the polyglyceryl fatty ester surfactant and the phosphate ester co-surfactant is selected in the range of 6:1 to 2:1, preferably in the range of 5:1 to 3:1.

5. The topical composition according to any one of claims 1 to 4, **characterized in that** the phosphate ester surfactant is a compound of formula (I) 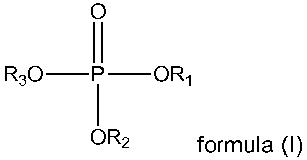 wherein
R₁, R₂ and R₃ are independently of each other hydrogen, C₁₋₂₂ alkyl, or a C₁₋₂₂ alkoxylated alkyl having 2 to 25 moles ethylene oxide, with the proviso that at least one of R₁, R₂ and R₃ is an alkyl or an alkoxylated alkyl having at least 6 alkyl carbons in said alkyl or alkoxylated alkyl group.

6. The topical composition according to claim 5, **characterized in that** the phosphate ester surfactant is potassium cetyl phosphate.

7. The topical composition according to any one of claims 1 to 6, **characterized in that** the polyglycerol fatty ester is selected from the group consisting of polyglyceryl-3-stearate and polyglyceryl-10-stearate.

8. The topical composition according to any one of claims 1 to 7, **characterized in that** the polyglyceryl fatty ester surfactant has an HLB of 5 or greater.

9. The topical composition according to claim 8, **characterized in that** the HLB is selected in range of 6 to 15, preferably in the range of 9 to 14.

10. The topical composition according to any one of claims 1 to 9, **characterized in that** the topical composition is in the form of a serum, an emulsion spray, a milk, a lotion or a cream.

11. Use of a phosphate ester surfactant to reduce the viscosity of a topical composition in the form of an O/W emulsion comprising an oil phase dispersed in an aqueous phase in the presence of a polyglyceryl fatty ester surfactant, **characterized in that** the polyglyceryl fatty ester surfactant is an ester of stearic acid or isostearic acid of formula (II) 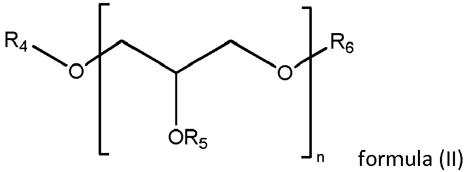 wherein R₄, R₅ and R₆ are independently of each other hydrogen, a stearoyl and/ or an isostearoyl group with the proviso that at least one of R₄, R₅ and R₆ is a stearoyl- and/ or an isostearoyl-group and n is an integer selected from the group of 2 to 10 and wherein the wt.-ratio between the polyglyceryl fatty ester surfactant and the phosphate ester co-surfactant is selected in the range of 8:1 to 1:1.

12. A method for reducing the viscosity of a topical composition in the form of an oil-in-water (O/W) emulsion comprising an oil phase dispersed in an aqueous phase in the presence of a polyglyceryl fatty ester surfactant, said method comprising the addition of a phosphate ester co-surfactant, **characterized in that** the polyglyceryl fatty ester surfactant is an ester of stearic acid or isostearic acid of formula (II) 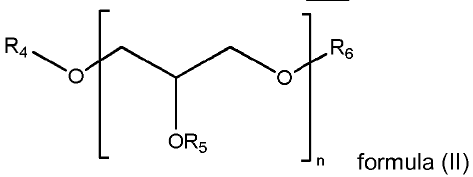 wherein R₄, R₅ and R₆ are independently of each other hydrogen, a stearoyl and/ or an isostearoyl group with the proviso that at least one of R₄, R₅ and R₆ is a stearoyl- and/ or an isostearoyl-group and n is an integer selected from the group of 2 to 10 and wherein the wt.-ratio between the polyglyceryl fatty ester surfactant and the phosphate ester co-surfactant is selected in the range of 8:1 to 1:1.

13. A method of preserving the long-term thermal storage stability of a topical composition in the form of an oil-in-water (O/W) emulsion comprising an oil phase dispersed in an aqueous phase in the presence of a polyglyceryl fatty ester surfactant, said method comprising the addition of a phosphate ester co-surfactant, **characterized in that** the polyglyceryl fatty ester surfactant is an ester of stearic acid or isostearic acid of formula (II) 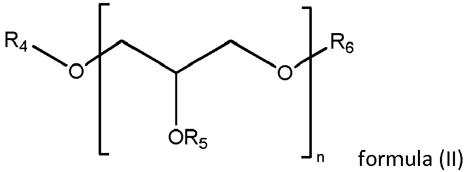 wherein R₄, R₅ and R₆ are independently of each other hydrogen, a stearoyl and/ or an isostearoyl group with the proviso that at least one of R₄, R₅ and R₆ is a stearoyl- and/ or an isostearoyl-group and n is an integer selected from the group of 2 to 10 and wherein the wt.-ratio between the polyglyceryl fatty ester surfactant and the phosphate ester co-surfactant is selected in the range of 8:1 to 1:1.

14. The use or the method according to any one of claims 11 to 13, wherein the ester of stearic acid or isostearic acid of formula (II) is selected from the group consisting of polyglyceryl-3-stearate, polyglyceryl-10-stearate polyglyceryl-2- diisostearate, polyglyceryl-6-distearate and polyglyceryl-10 diisostearate.

## Patentansprüche

1. Topische Zusammensetzung in Form einer Öl-in-Wasser(O/W)-Emulsion, umfassend eine in Gegenwart eines Polyglycerylfettester-Tensids und eines Phosphatester-Cotensids in einer wässrigen Phase dispergierte Ölphase, **dadurch gekennzeichnet, dass** es sich bei dem Polyglycerylfettester-Tensid um einen Monoester von Stearinsäure oder Isostearinsäure der Formel (II) 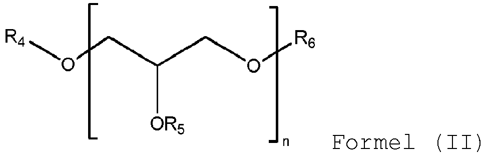 handelt, wobei
R₄, R₅ und R₆ unabhängig voneinander für Wasserstoff, eine Stearoyl- und/oder eine Isostearylgruppe stehen, mit der Maßgabe, dass eines von R₄, R₅ und R₆ für eine Stearoyl- und/oder eine Isostearylgruppe steht und die anderen für Wasserstoff stehen, und n für eine ganze Zahl, die aus 2 bis 10 ausgewählt ist, steht, und wobei das Gewichtsverhältnis zwischen dem Polyglycerylfettester-Tensid und dem Phosphatester-Cotensid im Bereich von 8:1 bis 1:1 ausgewählt ist.

2. Topische Zusammensetzung nach Anspruch 1, **dadurch gekennzeichnet, dass** die Menge des Polyglycerylfettester-Tensids im Bereich von 0,1 bis 10 Gew.-%, vorzugsweise im Bereich von 0,3 bis 5 Gew.-%, ganz besonders bevorzugt im Bereich von 0,5 bis 3 Gew.-%, bezogen auf das Gesamtgewicht der topischen Zusammensetzung, ausgewählt ist.

3. Topische Zusammensetzung nach Anspruch 1 oder 2, **dadurch gekennzeichnet, dass** die Menge des Phosphatester-Cotensids im Bereich von 0,01 bis 3 Gew.-%, vorzugsweise im Bereich von 0,05 bis 2 Gew.-%, ganz besonders bevorzugt im Bereich von 0,1 bis 1 Gew.-%, bezogen auf das Gesamtgewicht der topischen Zusammensetzung, ausgewählt ist.

4. Topische Zusammensetzung nach einem der Ansprüche 1 bis 3, **dadurch gekennzeichnet, dass** das Gewichtsverhältnis zwischen dem Polyglycerylfettester-Tensid und dem Phosphatester-Cotensid im Bereich von 6:1 bis 2:1, vorzugsweise im Bereich von 5:1 bis 3:1, ausgewählt ist.

5. Topische Zusammensetzung nach einem der Ansprüche 1 bis 4, **dadurch gekennzeichnet, dass** es sich bei dem Phosphatester-Tensid um eine Verbindung der Formel (I) 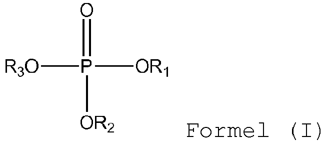 handelt, wobei
R₁, R₂ und R₃ unabhängig voneinander für Wasserstoff, C₁₋₂₂-Alkyl oder ein alkoxyliertes C₁₋₂₂-Alkyl mit 2 bis 25 mol Ethylenoxid stehen, mit der Maßgabe, dass mindestens eines von R₁, R₂ und R₃ für ein Alkyl oder ein alkoxyliertes Alkyl mit mindestens 6 Alkyl-Kohlenstoffatomen in der Alkyl- bzw. alkoxylierten Alkylgruppe steht.

6. Topische Zusammensetzung nach Anspruch 5, **dadurch gekennzeichnet, dass** es sich bei dem Phosphatester-Tensid um Kaliumcetylphosphat handelt.

7. Topische Zusammensetzung nach einem der Ansprüche 1 bis 6, **dadurch gekennzeichnet, dass** der Polyglycerinfettester aus der Gruppe bestehend aus Polyglyceryl-3-stearat und Polyglyceryl-10-stearat ausgewählt ist.

8. Topische Zusammensetzung nach einem der Ansprüche 1 bis 7, **dadurch gekennzeichnet, dass** das Polyglycerylfettester-Tensid einen HLB-Wert von 5 oder mehr aufweist.

9. Topische Zusammensetzung nach Anspruch 8, **dadurch gekennzeichnet, dass** der HLB-Wert im Bereich von 6 bis 15, vorzugsweise im Bereich von 9 bis 14, ausgewählt ist.

10. Topische Zusammensetzung nach einem der Ansprüche 1 bis 9, **dadurch gekennzeichnet, dass** die topische Zusammensetzung in Form eines Serums, eines Emulsionssprays, einer Milch, einer Lotion oder einer Creme vorliegt.

11. Verwendung eines Phosphatester-Tensids zur Verringerung der Viskosität einer topischen Zusammensetzung in Form einer O/W-Emulsion, umfassend eine in Gegenwart eines Polyglycerylfettester-Tensids in einer wässrigen Phase dispergierte Ölphase, **dadurch gekennzeichnet, dass** es sich bei dem Polyglycerylfettester-Tensid um einen Ester von Stearinsäure oder Isostearinsäure der Formel (II) 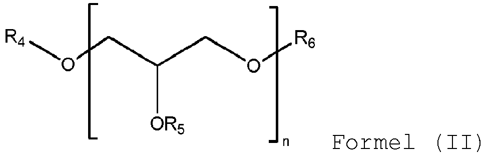 handelt, wobei
R₄, R₅ und R₆ unabhängig voneinander für Wasserstoff, eine Stearoyl- und/oder eine Isostearylgruppe stehen, mit der Maßgabe, dass mindestens eines von R₄, R₅ und R₆ für eine Stearoyl- und/oder eine Isostearylgruppe steht, und n für eine ganze Zahl, die aus der Gruppe von 2 bis 10 ausgewählt ist, steht, und wobei das Gewichtsverhältnis zwischen dem Polyglycerylfettester-Tensid und dem Phosphatester-Cotensid im Bereich von 8:1 bis 1:1 ausgewählt ist.

12. Verfahren zur Verringerung der Viskosität einer topischen Zusammensetzung in Form einer O/W-Emulsion, umfassend eine in Gegenwart eines Polyglycerylfettester-Tensids in einer wässrigen Phase dispergierte Ölphase, bei dem man ein Phosphatester-Cotensid zugibt, **dadurch gekennzeichnet, dass** es sich bei dem Polyglycerylfettester-Tensid um einen Ester von Stearinsäure oder Isostearinsäure der Formel (II) 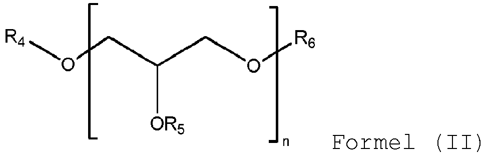 handelt, wobei
R₄, R₅ und R₆ unabhängig voneinander für Wasserstoff, eine Stearoyl- und/oder eine Isostearylgruppe stehen, mit der Maßgabe, dass mindestens eines von R₄, R₅ und R₆ für eine Stearoyl- und/oder eine Isostearylgruppe steht, und n für eine ganze Zahl, die aus der Gruppe von 2 bis 10 ausgewählt ist, steht, und wobei das Gewichtsverhältnis zwischen dem Polyglycerylfettester-Tensid und dem Phosphatester-Cotensid im Bereich von 8:1 bis 1:1 ausgewählt ist.

13. Verfahren zur Bewahrung der thermischen Langzeitlagerstabilität einer topischen Zusammensetzung in Form einer O/W-Emulsion, umfassend eine in Gegenwart eines Polyglycerylfettester-Tensids in einer wässrigen Phase dispergierte Ölphase, bei dem man ein Phosphatester-Cotensid zugibt, **dadurch gekennzeichnet, dass** es sich bei dem Polyglycerylfettester-Tensid um einen Ester von Stearinsäure oder Isostearinsäure der Formel (II) 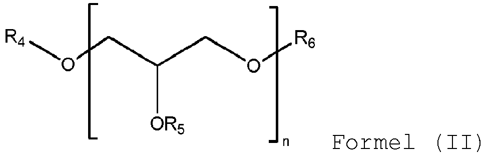 handelt, wobei
R₄, R₅ und R₆ unabhängig voneinander für Wasserstoff, eine Stearoyl- und/oder eine Isostearylgruppe stehen, mit der Maßgabe, dass mindestens eines von R₄, R₅ und R₆ für eine Stearoyl- und/oder eine Isostearylgruppe steht, und n für eine ganze Zahl, die aus der Gruppe von 2 bis 10 ausgewählt ist, steht, und wobei das Gewichtsverhältnis zwischen dem Polyglycerylfettester-Tensid und dem Phosphatester-Cotensid im Bereich von 8:1 bis 1:1 ausgewählt ist.

14. Verwendung bzw. Verfahren nach einem der Ansprüche 11 bis 13, wobei der Ester von Stearinsäure oder Isostearinsäure der Formel (II) aus der Gruppe bestehend aus Polyglyceryl-3-stearat, Polyglyceryl-10-stearat, Polyglyceryl-2-diisostearat, Polyglyceryl-6-distearat und Polyglyceryl-10-distearat ausgewählt ist.

## Revendications

1. Composition topique sous la forme d'une émulsion huile-dans-eau (H/E) comprenant une phase huileuse dispersée dans une phase aqueuse en présence d'un agent tensioactif à base d'un ester gras polyglycérylé et d'un co-agent tensioactif à base d'un ester de phosphate, **caractérisée en ce que** l'agent tensioactif à base d'un ester gras polyglycérylé est un monoester d'acide stéarique ou d'acide isostéarique de formule (II) 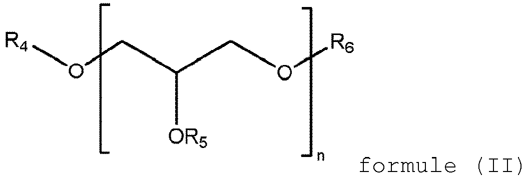 où
R₄, R₅ et R₆ sont indépendamment les uns des autres hydrogène, un groupement stéaroyle et/ou isostéaroyle, à condition que l'un parmi R₄, R₅ et R₆ soit un groupement stéaroyle et/ou isostéaroyle et que les autres soient hydrogène, et n est un nombre entier choisi parmi de 2 à 10, et où le rapport pondéral entre l'agent tensioactif à base d'un ester gras polyglycérylé et le co-agent tensioactif à base d'un ester de phosphate est choisi dans la plage allant de 8:1 à 1:1.

2. Composition topique selon la revendication 1, **caractérisée en ce que** la quantité de l'agent tensioactif à base d'un ester gras polyglycérylé est choisie dans la plage allant de 0,1 à 10% en poids, préférablement dans la plage allant de 0,3 à 5% en poids, tout préférablement dans la plage allant de 0,5 à 3% en poids, sur la base du poids total de la composition topique.

3. Composition topique selon la revendication 1 ou 2, **caractérisée en ce que** la quantité du co-agent tensioactif à base d'un ester de phosphate est choisie dans la plage allant de 0,01 à 3% en poids, préférablement dans la plage allant de 0,05 à 2% en poids, tout préférablement dans la plage allant de 0,1 à 1% en poids, sur la base du poids total de la composition topique.

4. Composition topique selon l'une quelconque des revendications 1 à 3, **caractérisée en ce que** le rapport pondéral entre l'agent tensioactif à base d'un ester gras polyglycérylé et le co-agent tensioactif à base d'un ester de phosphate est choisi dans la plage allant de 6:1 à 2:1, préférablement dans la plage allant de 5:1 à 3:1.

5. Composition topique selon l'une quelconque des revendications 1 à 4, **caractérisée en ce que** l'agent tensioactif à base d'un ester de phosphate est un composé de formule (I) 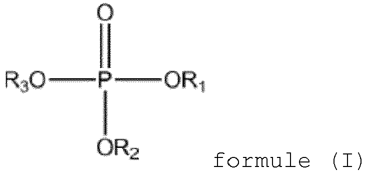 où
R₁, R₂ et R₃ sont indépendamment les uns des autres hydrogène, C₁₋₂₂-alkyle, ou C₁₋₂₂-alkyle alcoxylé ayant de 2 à 25 moles d'oxyde d'éthylène, à condition qu'au moins l'un parmi R₁, R₂ et R₃ soit un groupement alkyle ou alkyle alcoxylé ayant au moins 6 carbones d'alkyle dans ledit groupement alkyle ou alkyle alcoxylé.

6. Composition topique selon la revendication 5, **caractérisée en ce que** l'agent tensioactif à base d'un ester de phosphate est le cétyl phosphate de potassium.

7. Composition topique selon l'une quelconque des revendications 1 à 6, **caractérisée en ce que** l'ester gras de polyglycérol est choisi dans le groupe constitué par le polyglycéryl-3-stéarate et le polyglycéryl-10-stéarate.

8. Composition topique selon l'une quelconque des revendications 1 à 7, **caractérisée en ce que** l'agent tensioactif à base d'un ester gras polyglycérylé possède un HLB de 5 ou plus.

9. Composition topique selon la revendication 8, **caractérisée en ce que** le HLB est choisi dans la plage allant de 6 à 15, préférablement dans la plage allant de 9 à 14.

10. Composition topique selon l'une quelconque des revendications 1 à 9, **caractérisée en ce que** la composition topique se trouve sous la forme d'un sérum, d'une émulsion en spray, d'un lait, d'une lotion ou d'une crème.

11. Utilisation d'un agent tensioactif à base d'un ester de phosphate afin de réduire la viscosité d'une composition topique sous la forme d'une émulsion H/E comprenant une phase huileuse dispersée dans une phase aqueuse en présence d'un agent tensioactif à base d'un ester gras polyglycérylé, **caractérisée en ce que** l'agent tensioactif à base d'un ester gras polyglycérylé est un ester d'acide stéarique ou d'acide isostéarique de formule (II) 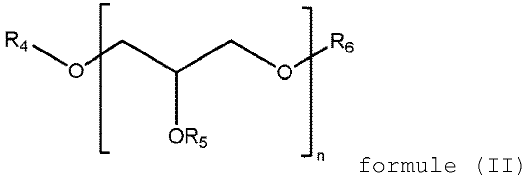 où
R₄, R₅ et R₆ sont indépendamment les uns des autres hydrogène, un groupement stéaroyle et/ou isostéaroyle, à condition qu'au moins l'un parmi R₄, R₅ et R₆ soit un groupement stéaroyle et/ou isostéaroyle, et n est un nombre entier choisi dans le groupe allant de 2 à 10, et où le rapport pondéral entre l'agent tensioactif à base d'un ester gras polyglycérylé et le co-agent tensioactif à base d'un ester de phosphate est choisi dans la plage allant de 8:1 à 1:1.

12. Méthode de réduction de la viscosité d'une composition topique sous la forme d'une émulsion huile-dans-eau (H/E) comprenant une phase huileuse dispersée dans une phase aqueuse en présence d'un agent tensioactif à base d'un ester gras polyglycérylé, ladite méthode comprenant l'addition d'un co-agent tensioactif à base d'un ester de phosphate, **caractérisée en ce que** l'agent tensioactif à base d'un ester gras polyglycérylé est un ester d'acide stéarique ou d'acide isostéarique de formule (II) 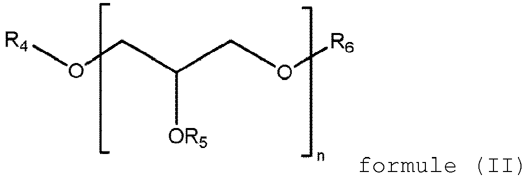 où
R₄, R₅ et R₆ sont indépendamment les uns des autres hydrogène, un groupement stéaroyle et/ou isostéaroyle, à condition qu'au moins l'un parmi R₄, R₅ et R₆ soit un groupement stéaroyle et/ou isostéaroyle, et n est un nombre entier choisi dans le groupe allant de 2 à 10, et où le rapport pondéral entre l'agent tensioactif à base d'un ester gras polyglycérylé et le co-agent tensioactif à base d'un ester de phosphate est choisi dans la plage allant de 8:1 à 1:1.

13. Méthode de conservation de la stabilité au stockage thermique à long terme d'une composition topique sous la forme d'une émulsion huile-dans-eau (H/E) comprenant une phase huileuse dispersée dans une phase aqueuse en présence d'un agent tensioactif à base d'un ester gras polyglycérylé, ladite méthode comprenant l'addition d'un co-agent tensioactif à base d'un ester de phosphate, **caractérisée en ce que** l'agent tensioactif à base d'un ester gras polyglycérylé est un ester d'acide stéarique ou d'acide isostéarique de formule (II) 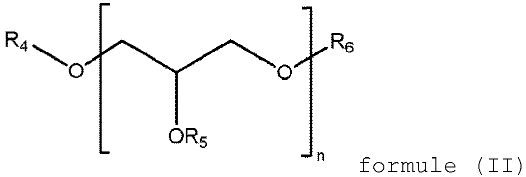 où
R₄, R₅ et R₆ sont indépendamment les uns des autres hydrogène, un groupement stéaroyle et/ou isostéaroyle, à condition qu'au moins l'un parmi R₄, R₅ et R₆ soit un groupement stéaroyle et/ou isostéaroyle, et n est un nombre entier choisi dans le groupe allant de 2 à 10, et où le rapport pondéral entre l'agent tensioactif à base d'un ester gras polyglycérylé et le co-agent tensioactif à base d'un ester de phosphate est choisi dans la plage allant de 8:1 à 1:1.

14. Utilisation ou méthode selon l'une quelconque des revendications 11 à 13, **caractérisée en ce que** l'ester d'acide stéarique ou d'acide isostéarique de formule (II) est choisi dans le groupe constitué par le polyglycéryl-3-stéarate, le polyglycéryl-10-stéarate, le polyglycéryl-2-diisostéarate, le polyglycéryl-6-distéarate et le polyglycéryl-10-diisostéarate.
